Europäisches Patentamt

European Patent Office

Office européen des brevets

(11) Veröffentlichungsnummer: **0 207 398**
A2

# (12) EUROPÄISCHE PATENTANMELDUNG

(21) Anmeldenummer: 86108431.7

(22) Anmeldetag: 20.06.86

(51) Int.Cl.⁴: **A 61 K 31/44**
//(A61K31/44, 31:365)

(30) Priorität: 02.07.85 DE 3523544

(43) Veröffentlichungstag der Anmeldung:
07.01.87 Patentblatt 87/2

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Gross, Rainer, Dr.
Platzhoffstrasse 23
D-5600 Wuppertal 1(DE)

(72) Erfinder: Schramm, Matthias, Dr.
Paffratherstrasse 38
D-5000 Köln 80(DE)

(54) Nifedipin Kombinationspräparat.

(57) Die vorliegende Erfindung betrifft ein festes Kombinationspräparat enthaltend Nifedipin und Isosorbid-5-mononitrat, welches zur Langzeitbehandlung von Herzerkrankungen, insbesondere von Erkrankungen der Coronarien und des Herzmuskels eingesetzt werden kann.

EP 0 207 398 A2

Croydon Printing Company Ltd.

Bayer Aktiengesellschaft        5190 Leverkusen, Bayerwerk
Konzernverwaltung RP            KS/Gh
Patentabteilung


Nifedipin Kombinationspräparat


Die vorliegende Erfindung betrifft ein festes Kombinationspräparat enthaltend Nifedipin und Isosorbid-5-mononitrat,
welches zur Langzeitbehandlung von Herzerkrankungen, insbesondere von Erkrankungen der Coronarien und des Herzmuskels eingesetzt werden kann.

Die Verbindung Nifedipin ist bekannt (Britisches Patent
1 173 862) und wird mit Erfolg eingesetzt bei der Therapie
der Hypertonie, der coronaren Herzkrankheit und der Herzinsuffizienz.

Die bekannte Verbindung Isosorbid-5-mononitrat (nachfolgend
IS-5-MN genannt) wirkt ebenfalls gefäßdilatierend, wobei
sich bei der Behandlung von Herzerkrankungen eine bevorzugte Wirkung an den Venen zeigt. Bei der Langzeit-Behandlung
von Herzerkrankungen mit Nitro-Präparaten zeigt sich häufig
eine unerwünschte Toleranz-Entwicklung, die eine Steigerung
der Dosierung erforderlich macht bzw. keine ausreichende
therapeutische Wirkung mehr zeigt.

Die Erfindung betrifft feste, oral applizierbare Arzneizubereitungen zur Verwendung bei der langzeitigen Bekämpfung
von Herzerkrankungen, insbesondere Erkrankungen der Coronarien
und des Herzmuskels, enthaltend 5 bis 30 mg des Wirkstoffs
Nifedipin und 10 bis 40 mg des Wirkstoffs Isosorbid-5-mono-
nitrat sowie gegebenenfalls übliche Hilfs- und Trägerstoffe.


Le A 23 855 -Ausland

Von besonderem Interesse sind solche Kombinationspräparate, welche 10 bis 30 mg Nifedipin und 10 bis 30 mg Isosorbid-5-mononitrat enthalten.

Überraschenderweise wird bei Anwendung der erfindungsgemäßen Kombination von Nifedipin und IS-5-MN die Toleranzentwicklung gegenüber der Nitrat-Komponente verhindert, was sich auch in Versuchen an isolierten Geweben nachweisen läßt. So läßt sich beispielsweise die Toleranzentwicklung von Nitraten durch Bestimmung ihrer Wirkung auf die Serotonininduzierte Kontraktion von Pulmonalvenen nachweisen (U. Borchard et al., Med. Klin. Sondernummer 1 (1985), 21- 23).

Setzt man in diesen Versuchen den Substanzeffekt direkt nach Substanzgabe als 100 %, so ist die zeitliche Abnahme dieses Effektes ein Maß für die Toleranzentwicklung. Nach fünf Stunden ergeben sich die folgenden Werte:

| Konzentration (g/ml) | | Wirkungsverlust nach 5 Stunden (%) |
|---|---|---|
| IS-5-MN | Nifedipin | |
| $10^{-4}$ | 0 | 42 |
| $3 \times 10^{-4}$ | 0 | 48 |
| 0 | $10^{-6}$ | 0 |
| 0 | $3 \times 10^{-6}$ | 0 |
| $10^{-4}$ | $10^{-6}$ | 7 |

Dieser unerwartete Befund ist insbesondere bei der notwendigen Langzeittherapie der genannten Krankheitsbilder von entscheidendem Vorteil.

Darüberhinaus versetzt vorliegende Erfindung den Fachmann in die Lage, den gleichen therapeutischen Effekt mit wesentlich reduzierter Dosierung der Einzelkomponenten der Wirk-

Le A 23 855

stoffe zu erreichen. Hierdurch werden ebenfalls unerwünschte Nebenwirkungen reduziert. Dies ist insbesondere bei der Langzeittherapie von großem Vorteil.

Die nachfolgenden Beispiele erläutern spezielle Ausführungsformen der erfindungsgemäßen Kombinationspräparate ohne beschränkende Wirkung zu haben.

Herstellungsbeispiele

Beispiel 1

10 g Nifedipin (mikrofein) werden mit 80 g einer Verreibung von IS-5-MN in Lactose (25 %ig), 41,4 g Maisstärke, 15 g Lactose und 20 g Avicel gemischt und mit einer Lösung aus 0,8 g Natriumlaurylsulfat und 12 g Polyvinylpyrrolidon (PVP 25) granuliert. Das Granulat wird getrocknet, gesiebt und nach Zusatz von 0,8 g Magnesiumstearat zu Tabletten von einem Gewicht von 180 mg verpreßt oder in Steckkapseln mit 180 mg Kapselinhalt abgefüllt.

Beispiel 2

15 g Nifedipin werden mit 20 g einer Verreibung von IS-5-MN in Lactose (50 %ig), 15 g mikrokristalliner Cellulose, 30 g Lactose und 16 g Maisstärke gemischt und mit einem Kleister aus 4,25 g Maisstärke granuliert. Das erhaltene Granulat 1 wird in Steckkapseln abgefüllt oder zu Tabletten, die 5, 10 oder 20 mg Nifedipin enthalten, verpreßt.

Beispiel 3

Das Granulat 1 gemäß Beispiel 2 wird unter Zusatz von 4 % quervernetztem Polyvinylpyrrolidon und 1 % Magnesiumstearat mit dem folgenden Granulat 2, welches Nifedipin in Kopräzipitatform enthält, vermischt und zu Tabletten verpreßt.

Le A 23 855

0207398

Herstellung des Granulates 2:
10 g Nifedipin und 40 g PVP 25 werden in 60 g Aceton gelöst. Diese Lösung wird mit einer Mischung von 105 g mikrokristalliner Cellulose, 20 g Maisstärke und 10 g quervernetztem Polyvinylpyrrolidon granuliert und vor der Zusammenmischung mit Granulat 1 im Vakuum getrocknet.

Beispiel 4 (Herstellung von Granulat 1)
20 g Nifedipin werden mit 40 g einer Verreibung von IS-5-MN in Lactose (25 %ig), 10 g mikrokristalliner Cellulose und 20 g Maisstärke gemischt und mit einem Kleister aus 6 g Maisstärke granuliert. Anschließend wird dieses Granulat 1 mit dem Kopräzipitat-Granulat des Beispiels 3 vermischt und zu Tabletten verpreßt.

Le A 23 855

## Patentansprüche

1. Feste, oral applizierbare Arzneizubereitung enthaltend 5 bis 30 mg des Wirkstoffs Nifedipin und 10 bis 40 mg des Wirkstoffs Isosorbid-5-mononitrat.

2. Arzneizubereitungen gemäß Anspruch 1 enthaltend 10 bis 30 mg Nifedipin und 10 bis 30 mg Isosorbid-5-mononitrat.

3. Arzneizubereitungen gemäß Ansprüchen 1 und 2, dadurch gekennzeichnet, daß vom Gesamtanteil des Nifedipins 5 bis 10 mg pro Zubereitung in Kopräzipitatform vorliegen.

4. Verfahren zur Herstellung von Arzneizubereitungen gemäß Ansprüchen 1 bis 3, dadurch gekennzeichnet, daß man Nifedipin und Isosorbid-5-mononitrat gegebenenfalls unter Verwendung von üblichen Hilfs- und Trägerstoffen in eine geeignete Applikationsform überführt.

5. Verwendung von Arzneizubereitungen gemäß Ansprüchen 1 bis 3 zur Herstellung von Arzneimitteln mit Kreislaufwirkung.

6. Verwendung von Arzneizubereitungen gemäß Ansprüchen 1 bis 3 zur Bekämpfung von coronaren Herzkrankheiten, Herzinsuffizienz und Hypertonie.

Le A 23 855